# EUROPÄISCHE PATENTSCHRIFT

(11) **EP 0 894 488 B1**
(45) Veröffentlichungstag und Bekanntmachung des Hinweises auf die Patenterteilung: **01.03.2006**
(21) Anmeldenummer: 98113855.5
(22) Anmeldetag: 24.07.1998
(51) Int. Cl.: A61K 6/083, C01G 57/00

(54) **Dentalmasse mit neuartigen Füllstoffen**
Dental material with new filler
Matériau dentaire avec des charges nouvelles

(30) Priorität: 28.07.1997 DE 19732356
(43) Veröffentlichungstag der Anmeldung: 03.02.1999
(73) Patentinhaber: VOCO GmbH, 27472 Cuxhaven (DE)
(72) Erfinder: Maletz, Reinhard, Dr., 27472 Cuxhaven (DE); Danebrock, Martin, Dr., 27472 Cuxhaven (DE); Plaumann, Manfred Thomas, 27476 Cuxhaven (DE)

(56) Entgegenhaltungen:
- EP-A- 0 238 025
- DE-A- 2 347 591
- DE-A- 3 421 155

## Beschreibung

Die Erfindung betrifft Dentalmaterialien wie Composite, Compomere, Glas Ionomer Zemente, Carboxylatzemente oder Phosphatzemente, die neben üblichen Füllstoffen und Hilfsmitteln Mischkristalle als Füllstoff enthalten. Die Mischkristalle haben eine entsprechend den Ansprüchen formulierte Zusammensetzung. Die Mischkristalle stellen als Füllstoff die überraschenderweise ausgezeichneten physikalischen Eigenschaften, wie Abrasionsfestigkeit, Druckfestigkeit, Röntgenopazität, visuelle Transluzenz sowie physikalische und chemische Resistenz des Dentalmaterials sicher.

Gemische oder Mischungen bestehen aus zwei oder mehreren verschiedenen Substanzen, die keine chemische Reaktion miteinander eingehen und sich durch physikalische Trennverfahren in ihre Komponenten zerlegen lassen. Feste Lösungen oder Mischkristalle bezeichnen Verbindungen, die aus zwei oder mehreren Komponenten homogen aufgebaut sind. Die gelösten Komponenten können entweder Gitterplätze im Kristallgitter des Lösungsmittels besetzen oder zwischen den Gitterbausteinen eingelagert sein. Mischkristalle sind Verbindungen, deren Plätze im Kristallgitter durch Atome oder Ionen von zwei oder mehreren verschiedenen Elementen oder Verbindungen besetzt sind. Sie zeigen gegenüber den sie aufbauenden Einzelbestandteilen makroskopisch unterschiedliche Eigenschaften. Die Herstellung von Mischkristallen, insbesondere von Schwermetallsalzen, ist in gängigen chemischen Lehrbüchern offenbart (s. z.B. Simon, Angewandte Chemie, 95 (1983))

Compositematerialien sind seit 1962 als Zahnrestaurationsmaterialien bekannt (Bowen, R. L.; US 3066112). Die Glas lonomer Zemente wurden in den 60er Jahren von Wilson & Kent eingeführt (Wilson, A. D.; J Dent Res 75 (10), 1723 (1996)). Die Dentalmaterialgruppe der Compomere ist beispielsweise durch die Patentschrift EP 0219058 offenbart. 1968 erwähnte D. C. Smith eine neue Gruppe Dentalzemente, die Carboxylatzemente (Smith. D. C.; Britisch Dental J 125, 381 (1968)). Die Phosphatzemente sind seit über 100 Jahren bekannt und noch heute als Dentalmassen gebräuchlich.

Zur Gewährleistung der anerkannten Materialeigenschaften dieser Dentalmassen werden eine Reihe verschiedene, vor allem anorganische Füllstoffe zugesetzt. Als wesentlich für die Qualität eines Dentalmaterials sind die Füllstoffeigenschaften zu bewerten. So üben die Partikelgröße, die Größenverteilung, die Partikeloberfläche, die chemische Zusammensetzung, die optischen Eigenschaften, die Röntgenopazität, die Bindungsfähigkeit zur Polymermatrix und der Gehalt an Füllmaterial einen entscheidenden Einfluß auf das Dentalmaterial aus. Zu beachten ist dabei, daß die Charakteristika der Füllstoffe sich untereinander beeinflussen können. Zur Optimierung des einen Parameters sind somit auch die anderen Einflußgrößen zu berücksichtigen. So haben beispielsweise zunehmende Füllstoffanteile eine zunehmende visuelle Opazität, eine reduzierte Transluzenz und eine begrenzte Aushärtungstiefe bei der lichtinitiierten Polymerisation zur Folge. Bei der Wahl entsprechender Füllstoffe für Dentalmassen ist somit eine exakte Abwägung notwendig, um die verschiedenen physikalischen oder chemischen Materialeigenschaften des Dentalmaterials zu gewährleisten. So muß bei den Materialien zwischen Transluzenz und Opazität, zwischen Abrasion und Polierbarkeit, zwischen Festigkeit, Fließfähigkeit und Stopfbarkeit, zwischen Haftung am Instrument und Haftung an der Zahnsubstanz, zwischen einer schnellen Abbindezeit und einer effektiven Verarbeitungszeit abgewogen werden.

Aus der Patentschrift DE 2403211 ist bekannt, daß sogenannte Mikrofüllstoffe in Compositen zu einer guten Druckfestigkeit, einem gutem Abrasionsverhalten und einem thermischen Expansionsverhalten ähnlich der Zahnsubstanz führen. Nachteilig wirkt sich dabei jedoch die schlechte Anbindung der Füllpartikel an die Harzmatrix aus, was u.a. auch zu einer Verschlechterung der Viskositätseinstellung führt.

Einen umfangreichen Überblick über anorganische Füllstoffe in Dentalmaterialien liefern die Patentschriften EP 189540, EP 159887, EP 473048 und EP 238025 sowie die darin zitierten Druckschriften. In den Patentschriften werden u.a. auch Verbindungen von Metallen mit einer hohen Elementnummer wie Barium, Strontium, Zirkonium, Yttrium und den Seltenerdmetallen offenbart. Danach erweisen sich vor allem die Fluoride dieser Metalle als scheinbar vorteilhaft für die Erzielung einiger erstrebenswerter Eigenschaften von Dentalmaterialien. Nachteilig wirkt sich bei diesen Fluoridverbindungen jedoch deren fehlende Anbindungsmöglichkeit an die Matrix des Dentalmaterials aus. Die Anbindung glasartiger Füllstoffe an die Polymermatrix von Dentalmassen gelingt mittels der Silanisierung über Hydroxyl- bzw. Oxygruppen der Glaspartikel. Fluoridverbindungen sind bekanntermaßen nicht silanisierbar und bewirken daher im ausgehärteten Füllmaterial eine unzureichende physikalische und chemische Resistenz.

Die Dauerhaftigkeit zahnärztlicher Füllungswerkstoffe im Mund ist weitgehend durch chemische, physikalische und biologische Angriffe bestimmt, deren Folgen als Verfärbung, Erweichung des Materials mit nachfolgendem Substanzverlust sowie der Gefahr von Sekundärkaries erkennbar werden. Insbesondere die Diffusion von Flüssigkeiten wie Speichel, Wasser, Alkohol oder Säuren führt zu Verfärbungen und zur Spannungskorrosion an der Grenzfläche zwischen Füllstoffteilchen und Kunststoffmatrix und der Erweichung der Matrix. Diese Nachteile, mangelnde physikalische und chemische Resistenz der bekannten Dentalmaterialien, gilt es zu vermeiden

Wenngleich aus dem Stand der Technik hervorgeht, daß Fluoride als auch Oxide der Seltenerdmetalle, Yttrium, Scandium, Zirkonium oder Strontium als Füllstoffe für Dentalmassen bekannt sind, um beispielsweise die Röntgenopazität zu erhöhen, so sollte der Anteil an Schwermetallen in dentalen Materialien jedoch begrenzt werden. Für diese Begrenzung sind die o.g. Gründe sowie zunehmend toxikologische Bedenken ausschlaggebend.

Der Erfindung liegt die Aufgabe zugrunde, aufbauend auf den Vorteilsmerkmalen der bekannten Dentalmaterialien die physikalischen Eigenschaften der Dentalmaterialien wesentlich zu verbessern und die jeweiligen Materialnachteile zu vermeiden. Die Aufgabe wird erfindungsgemäß durch Dentalmaterialien entsprechend den Ansprüchen 1 bis 3 gelöst, indem die Dentalmassen als Füllstoff Mischkristalle der in den Ansprüchen formulierten Zusammensetzung beinhalten. Die Eigenschaften der erfindungsgemäßen Dentalmaterialien sind aufgrund des Füllstoffes somit über einen weiten Bereich entsprechend den jeweiligen Anwendungserfordernissen variierbar.

Durch Verwendung der erfindungsgemäßen Mischkristalle kann eine definierte Menge an oxidischen Fehlstellen generiert werden, die beispielsweise eine Silanisierung und somit eine Anbindung an die Kunststoffmatrix ermöglicht. Damit wird neben den bekannten vorteilhaften Eigenschaften, wie kariosstatische Wirkung aufgrund der Fluoridabgabe, eine physikalische und chemische Resistenz der Dentalmaterialien geschaffen.

Durch das Vorliegen zweier unterschiedlicher Schwermetalle in den erfindungsgemäßen Dentalmaterialien ergeben sich verschiedenen K_{α}-Röntgenabsorptionslinien. Dadurch wird eine effektivere Röntgenabsorption und damit eine optimale Röntgenopazität erreicht. Bei vermindertem Schwermetallanteil wird der gleiche Aluminium-Standardreferenzwert (% Al gem. ISO 9917) der Röntgenopazität erreicht. Bei verbesserter Röntgenopazität kann somit gleichzeitig der Schwermetallanteil minimiert und die Bedenken gegen Schwermetallbelastungen verringert werden.

Überraschenderweise zeigt sich, daß die erfindungsgemäßen Dentalmassen hervorragende physikalische Eigenschaften aufweisen, wie beispielsweise Abrasions- und Druckfestigkeit, Röntgenopazität, visuelle Transluzenz sowie physikalische und chemische Resistenz.

Die Erfindung soll anhand nachstehender Beispiele näher erläutert werden.

### Beispiel 1:

Es werden Pasten folgender Zusammensetzung entsprechend den bekannten Herstellungsrichtlinien hergestellt:

| Bestandteile | **Paste A** [Gew.%] | **Paste B** [Gew.%] | **Paste C** [Gew.%] |
|---|---|---|---|
| silanisierte pyrogene Kieselsäure | 5 | 5 | 5 |
| silanisiertes Dentalglas | 28 | 66 | 28 |
| Campherchinon | 0,1 | 0,1 | 0,1 |
| N,N-Dimethylaminoethylmethacrylat | 1,5 | 1,5 | 1,5 |
| Bis-GMA | 13,6 | 13,6 | 13,6 |
| TEGDMA | 13,6 | 13,6 | 13,6 |
| Farbpigmente und Stabilisatoren | 0,2 | 0,2 | 0,2 |
| Füllstoff YF₃ | 38 | - | - |
| Füllstoff [La_{0.56}Y_{0,44}]OF | - | - | 38 |

Paste C entspricht einer beispielhaften, erfindungsgemäßen Zusammensetzung. Paste A und B dienen zum Vergleich, indem Paste A Yttriumfluorid und Paste B keinen zusätzlichen Füllstoff enthält.
Anschließend werden mit den ausgehärteten Füllmaterialien A, B und C die folgenden Eigenschaften nach standardisierten Meßverfahren untersucht. Beispielsweise ist Aluminium als Standardreferenz für die Röntgenopazität gebräuchlich (ISO 9917). Die Röntgenopazität einer 1 mm dicken Dentinschicht entspricht einer 1 mm dicken Aluminiumschicht (100% Al). Die Röntgenopazität einer 1 mm dicken Schmelzschicht ist äquivalent einer 2 mm dicken Aluminiumschicht (200% Al). Die effektive Röntgenopazität eines Dentalmaterials liegt somit vereinbarungsgemäß oberhalb 200% Al. Es ergeben sich folgende, in der Tabelle dargestellten, Resultate.

| | Schichtdicke [mm] | Röntgenopazität [% Al] | Transluzenz | Druck festigkeit [MPa] | Biegefestigkeit [MPa] | Abrasion in 5 Min. [µm] |
|---|---|---|---|---|---|---|
| Paste A | 8 | 180 | 0,29 | 348 | 85 | 110 |
| Paste B | 8 | 45 | 0,31 | 352 | 91 | 89 |
| **Paste C** | **8** | **260** | **0,30** | **368** | **98** | **62** |

### Beispiel 2:

In einer weiteren Untersuchung wird ein Dentalmaterial der Zusammensetzung C des Beispiels 1 über einen längeren Zeitraum bei 37°C jeweils in Wasser, Ethanol und 10%ige Salzsäure gelagert. Anschließend erfolgt eine Prüfung der Wasseraufnahme und Löslichkeit entsprechend der Norm EN ISO 10477 bzw. der darin festgelegten Prüfverfahren. Aus der Untersuchung ergeben sich folgende Ergebnisse:

| | | Wasser bzw. Lösungsmittelaufnahme [µg/mm³] | Löslichkeit [µg/mm³] |
|---|---|---|---|
| 1 Tag Lagerung in | Wasser | 0,5 | - |
| | Ethanol | 0,8 | 0,2 |
| | 10%ige Salzsäure | 0,8 | 0,5 |
| 7 Tage Lagerung in | Wasser | 5,3 | 0,1 |
| | Ethanol | 6,5 | 0,9 |
| | 10%ige Salzsäure | 8,0 | 1,1 |
| 14 Tage Lagerung in | Wasser | 6,9 | 0,2 |
| | Ethanol | 8,1 | 1,1 |
| | 10%ige Salzsäure | 10,2 | 1,0 |
| maximal Wert nach EN ISO 10477 | | 32 | 5 |

Die Beispiele verdeutlichen die vorteilhaften Eigenschaften der erfindungsgemäßen Dentalmaterialien mit Mischkristallen als Füllstoff.

## Patentansprüche

1. Polymerisierbares und/oder durch eine Salzreaktion aushärtendes Dentalmaterial, **dadurch gekennzeichnet, dass** es als Füllstoff Mischkristalle der Zusammensetzung
[M₁₋ₙM'ₙ]ₐ[X₃₋ₘZ₂ₘ]_{b}
enthält, wobei
M und M' ein Seltenerdmetall (Elemente 57 bis 71), Yttrium, Scandium, Zirkonium oder Strontium ist und
M ungleich M',
X ein Chalkogenid
und Z ein Halogenid ist und
wobei die stöchiometrische Zahl n Werte zwischen 0 und 1, die von n unabhängige stöchiometrische Zahl m Werte größer 0 bis einschließlich 1 und die stöchiometrischen Zahlen a und b ganzzahlige Werte des entsprechenden Valenzausgleiches annehmen.

2. Dentalmaterial nach Anspruch 1, **dadurch gekennzeichnet, dass** X ein Oxid, Sulfid oder Selenid und Z ein Fluorid, Chlorid oder Bromid ist.

3. Dentalmaterial nach einem der Ansprüche 1 oder 2, **dadurch gekennzeichnet, dass** der Anteil an Mischkristall-Füllstoff zwischen 2 und 90 Gew.% der Gesamtmasse des Dentalmaterials liegt.

## Claims

1. Polymerizable dental material and/or dental material which can be cured by a salt reaction, **characterized in that** it comprises, as filler, mixed crystals of the composition
[M₁₋ₙM'ₙ]ₐ[X₃₋ₘZ₂ₘ]_{b}
in which
M and M' are a rare earth metal (elements 57 to 71), yttrium, scandium, zirconium or strontium and
M is other than M',
X is a chalcogenide,
and Z is a halide, and
in which the stoichiometric figure n assumes values between 0 and 1, the stoichiometric figure m, which is independent of n, assumes values of greater than 0 up to 1 inclusive and the stoichiometric figures a and b assume integral values of the appropriate valency compensation.

2. Dental material according to Claim 1, **characterized in that** X is an oxide, sulphide or selenide and Z is a fluoride, chloride or bromide.

3. Dental material according to either of Claims 1 and 2, **characterized in that** the proportion of mixed crystal filler is between 2 and 90% by weight of the total weight of the dental material.

## Revendications

1. Matériau dentaire polymérisable et/ou durcissant par une réaction saline, **caractérisé en ce qu'**il contient, comme charge, des cristaux mixtes présentant la composition
[M₁-M'ₙ]ₐ[X₃₋ₘZ₂ₘ]_{b} où
M et M' représentent un métal de terre rare (éléments 57 à 71), l'yttrium, le scandium, le zirconium ou le strontium et
M est différent de M'
X représente un chalcogénure et
Z représente un halogénure et
le nombre stoechiométrique n présente des valeurs entre 0 et 1, le nombre stoechiométrique m indépendant de n présente des valeurs supérieures à 0 jusqu'à 1 compris et les nombres stoechiométriques a et b présentent des valeurs entières de l'équilibre de valence correspondant.

2. Matériau dentaire selon la revendication 1, **caractérisé en ce que** X représente un oxyde, un sulfure ou un séléniure et Z représente un fluorure, un chlorure ou un bromure.

3. Matériau dentaire selon l'une quelconque des revendications 1 ou 2, **caractérisé en ce que** la proportion de charge de cristaux mixtes est comprise entre 2 et 90% en poids de la masse totale du matériau dentaire.
